# EUROPEAN PATENT SPECIFICATION

(11) **EP 4 138 798 B1**
(45) Date of publication and mention of the grant of the patent: **09.07.2025**
(21) Application number: 21722978.0
(22) Date of filing: 20.04.2021
(51) Int. Cl.: A61K 31/07, A61P 11/00

(54) **TREATMENT OF PULMONARY FIBROSIS**
BEHANDLUNG VON LUNGENFIBROSE
TRAITEMENT DE LA FIBROSE PULMONAIRE

(30) Priority: 20.04.2020 GB 202005727; 23.03.2021 GB 202104041
(43) Date of publication of application: 01.03.2023
(73) Proprietor: Regenall Limited, London W1G 9QJ (GB)
(72) Inventor: NOORDEEN, Mohamed Hamza, London Greater London W1G 9QJ (GB)
(74) Representative: Reddie & Grose LLP
(86) International application number: PCT/GB2021/050948
(87) International publication number: WO 2021/214451

(56) References cited:
- EP-A1- 2 258 395
- EP-A1- 2 420 228
- EP-A2- 0 352 412
- WO-A1-2020/052742
- ES-A1- 2 387 109
- US-A1- 2019 343 764
- DYLER NICOLE: "Inhalable Vitamins Make Pill-Popping A Quaint 20th-Century Ritual", POPULAR SCIENCE, 28 October 2010 (2010-10-28), XP093127211, Retrieved from the Internet <URL:https://popsci.com.au/files/2010/10/inhalable-vitamins-make-pill-popping-a-quaint-20th-century-ritual/> [retrieved on 20240205]

## Description

This invention relates to compounds and compositions for use in the treatment of pulmonary fibrosis, including pulmonary fibrosis caused by respiratory infection. Methods of treatment of pulmonary fibrosis using the compounds are also described. The references to methods of treatment by therapy of this description are to be interpreted as references to compounds, pharmaceutical compositions and medicaments of the present invention for use in those methods.

Damage to tissues can result from various stimuli, including infections, autoimmune reactions, allergic responses, toxins, radiation and mechanical injury. The repair process typically involves two distinct phases: a regenerative phase, in which injured cells are replaced by cells of the same type, leaving no lasting evidence of damage; and a phase known as fibrosis, in which connective tissue replaces normal parenchymal tissue. Although initially beneficial, the repair process becomes pathogenic when it is not controlled appropriately, resulting in substantial deposition of extracellular matrix (ECM) components in which normal tissue is replaced with permanent scar tissue. In some diseases, extensive tissue remodelling and fibrosis can ultimately lead to organ failure and death.

Fibrosis is initiated when immune cells, such as macrophages, release soluble factors (such as TGF-β) that stimulate fibroblasts. These pro-fibrotic factors initiate signal transduction pathways, such as the AKT/mTOR and SMAD pathways, that ultimately lead to the proliferation and activation of fibroblasts, which deposit extracellular matrix into the surrounding connective tissue. ECM synthesis and degradation is tightly regulated, ensuring maintenance of normal tissue architecture. However, this process can lead to a progressive irreversible fibrotic response if tissue injury is severe or repetitive, or if the wound healing response itself becomes deregulated.

The key cellular mediator of fibrosis is the myofibroblast, which when activated serves as the primary collagen-producing cell. Myofibroblasts are generated from a variety of sources including resident mesenchymal cells, epithelial and endothelial cells in processes termed epithelial/endothelial-mesenchymal transition, as well as from circulating fibroblast-like cells called fibrocytes that are derived from bone-marrow stem cells. Myofibroblasts are activated by a variety of mechanisms, including paracrine signals derived from lymphocytes and macrophages, autocrine factors secreted by myofibroblasts, and pathogen-associated molecular patterns (PAMPS) produced by pathogenic organisms that interact with pattern recognition receptors on fibroblasts. Cytokines (IL-13, IL-21, TGF-β1), chemokines (MCP-1, MIP-1β), angiogenic factors (VEGF), growth factors (PDGF), peroxisome proliferator-activated receptors (PPARs), acute phase proteins (SAP), caspases, and components of the renin-angiotensin-aldosterone system (ANG II) have been identified as important regulators of fibrosis.

In pulmonary fibrosis, gradual exchange of normal lung parenchyma with fibrotic tissue causes an irreversible decrease in oxygen diffusion capacity as the lungs become scarred over time. Symptoms of pulmonary fibrosis include shortness of breath (particularly with exertion), chronic dry, hacking coughing, fatigue and weakness, chest discomfort including chest pain, loss of appetite and rapid weight loss. Complications may include pulmonary hypertension, respiratory failure, pneumothorax, and lung cancer.

Pulmonary fibrosis may be a secondary effect of other diseases or conditions (many of which are classified as interstitial lung diseases), including: infections, autoimmune diseases, connective tissue diseases, other diseases involving connective tissue, certain medications, radiation therapy, or inhalation of environmental and occupational pollutants. Cigarette smoking can increase the risk or make the illness worse. There have been reports of development of pulmonary fibrosis in patients infected with Severe acute respiratory syndrome coronavirus 2 (SARS-CoV-2), the causative agent of coronavirus disease 2019 (COVID-19) (see, for example, Shi et al., Lancet Infect Dis 2020;20: 425-34; see also Wadman et al., "How does coronavirus kill? Clinicians trace a ferocious rampage through the body, from brain to toes", Science, Apr. 17, 2020, doi:10.1126/science.abc3208).

Pulmonary fibrosis can also appear, however, without any known cause. Idiopathic pulmonary fibrosis (IPF) is the most common type of pulmonary fibrosis. About 5 million people are affected globally, with those in their 60s and 70s most commonly affected. Recent evidence supports the hypothesis that virus infection could play a key role in pathogenesis of IPF (Sheng et al "Viral Infection Increases the Risk of Idiopathic Pulmonary Fibrosis", Chest Journal (2019); online article: doi.org/10.1016/j.chest.2019.10.032). The authors conclude that the presence of persistent or chronic viral infections, including Epstein-Barr virus (EBV), cytomegalovirus (CMV), human herpesvirus 7 (HHV-7), and human herpesvirus 8 (HHV-8), significantly increases the risk of developing IPF (but not exacerbation of IPF), implying that viral infection could be a potential risk factor for IPF. There is also evidence which points to a genetic predisposition to IPF in a subset of patients. For example, a mutation in surfactant protein C (SP-C) has been found to exist in some families with a history of pulmonary fibrosis. Autosomal dominant mutations in the TERC or TERT genes, which encode telomerase, have been identified in about 15 percent of pulmonary fibrosis patients.

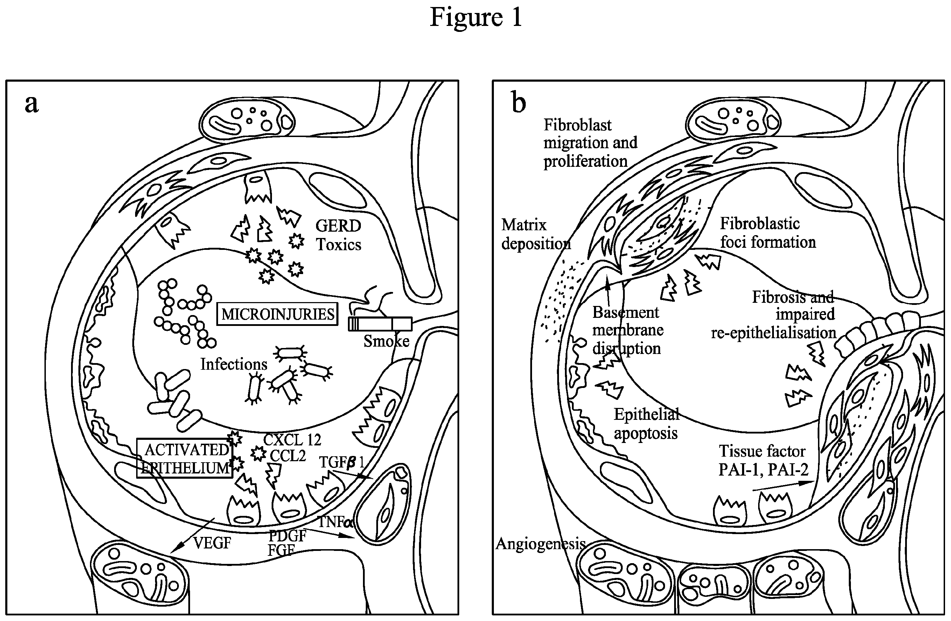

The factors driving the course of pulmonary fibrosis are not definitively identified. However, repeated microinjury to alveolar epithelial tissues is considered to be the first trigger of an aberrant repair process in which several lung cells develop abnormal behaviours that promote the fibrotic process. A dysfunctional, ageing lung epithelium exposed to recurrent microinjuries leads to defective attempts of regeneration and aberrant epithelial-mesenchymal crosstalk, creating an imbalance between profibrotic and antifibrotic mediators. Environments supportive of exaggerated fibroblast and myofibroblast activity are maintained, and the normal repair mechanisms are replaced with chronic fibrosis. Myofibroblasts, considered the effector cells of fibrogenesis, synthesise an abnormally stiff extracellular matrix. Mechanisms of pulmonary fibrosis are reviewed by Kinoshita and Goto (Int. J. Mol. Sci. 2019, 20, 1461; see Figure 1), and Sgalla et al (Respiratory Research (2018) 19:32). A schematic view of IPF pathogensis is provided in Figure 1 below (adapted from Figure 1 of Sgalla *et al*)*.*

There is no known cure for pulmonary fibrosis. IPF is a progressive disease with a 5-year survival rate of only 20%, reflecting the lack of effective therapies. Treatment is aimed at improving symptoms, and may include oxygen therapy and pulmonary rehabilitation. Certain medications may be used to try to slow the worsening of scarring. Immune suppressive agents, such as corticosteroids, may be used to decrease lung inflammation and subsequent scarring. However, responses to treatment are variable. Anti-inflammatory agents also have only limited success in reducing the fibrotic process. Therapeutic reagents pirfenidone and nintedanib were developed to slow the progression of pulmonary fibrosis. Whilst these agents are effective and well-tolerated (Hughes et al., "Real World Experiences: Pirfenidone and Nintedanib are Effective and Well Tolerated Treatments for Idiopathic Pulmonary Fibrosis", J Clin Med. 2016 Sep; 5(9): 78), they do not improve lung function and patients often remain with poor pulmonary function. Lung transplantation may be the only therapeutic option available in severe cases. European patent application 2258395 A1 describes a substance transfer carrier to an extracellular matrix (ECM) producing cell in the lung, comprising a retinoid as a targeting agent, and a therapeutic agent for treating pulmonary fibrosis. European patent application 0352412 A2 describes an inhalation aerosol comprising vitamin A for delivery to the mucus membrane of the respiratory tract.

An adequate vitamin A intake is required in early lung development, alveolar formation, tissue maintenance and regeneration. Timoneda et al. ("Vitamin A Deficiency and the Lung", Nutrients 2018; 10, 1132) review vitamin A deficiency (VAD) and the lung. Chronic VAD has been associated with histopathological changes in the pulmonary epithelial lining that disrupt the normal lung physiology, predisposing to severe tissue dysfunction and respiratory diseases. In addition, there are important alterations of the structure and composition of extracellular matrix (ECM) with thickening of the alveolar basement membrane (BM) and ectopic deposition of collagen I.

Pulmonary fibrosis is characterized by a replacement of normal lung parenchyma with fibrotic tissue accompanied by inflammation and excessive collagen deposition. The most prominent characteristic in the pathogenesis of lung fibrosis is persistent alveolitis, accumulation of myofibroblasts and the deposition of excessive amounts of ECM. Myofibroblasts (fibroblasts that express some features of muscle differentiation) are derived from resident mesenchymal cells, bone marrow progenitors (fibrocytes) and epithelial cells that have undergone epithelial-mesenchymal transition (EMT). One of the primary functions of the ECM is to maintain tissue integrity and homeostasis of multicellular organisms and it plays an important role in regulating alveolarization, tissue repair and remodelling in pulmonary tissue. Therefore, changes in the structure or composition of the ECM can induce alterations in cell and organ responses, leading to the development or progression of disease.

Retinoid signalling participates in the expression of ECM proteins, both directly (acting on their gene promoters) and indirectly (modifying the expression of profibrotic factors), and also affects the expression of cell membrane ECM receptors. Consequently, altered retinoid signalling induces changes in ECM/BM ultrastructure which are associated with fibrogenic activation in different organs and deterioration of tissue parenchyma. This can contribute to the disorders induced by VAD in organs and tissues.

Timoneda *et al.* (*supra*) report that, in an experimental model of chronic VAD rats, a thickening of the alveolar BM with an increase in the total amount of both type I and type IV collagens and a deposition of ectopic collagen fibrils in the BM was observed. The authors note that the mechanism through which VAD alters ECM is not clearly established, but explain that: *"an alteration of the TGF-β1*/*Smad3 signalling pathway has been considered to play a central role and is associated with lung fibrosis. Among the different possibilities suggested, an alteration in the transforming growth factor-β1 (TGF- β1)*/*Smad3 signalling pathway has been considered to play a central role, and is associated with lung fibrosis. TGF- β1 via the Smad signalling pathway can upregulate the expression of several collagens, and also via non-Smad signalling can activate the expression of other ECM molecules and its composition. Additionally, TGF- β1 is an inducer of EMT in alveolar epithelial cells, which has been suggested as an early event in the development of pulmonary fibrosis. Moreover, TGF- β1, via an integration of the Smad3 and STAT3 signalling pathways stimulates the connective tissue growth factor (CTGF), a central mediator of ECM production. In agreement, increased levels of TGF- β1 have been found in VAD tissues such as kidney, lung and aorta.*"

Although Timoneda *et al* report that most of the VAD-induced alterations of ECM are reversed by RA, and that RA exhibits anti-proliferative, anti-inflammatory, anti-migratory and anti-fibrogenic activities and ameliorates bleomycin-induced lung fibrosis by downregulating the TGF-β1/Smad3 signalling pathway in rats, there is no suggestion in this document regarding an effective treatment of pulmonary fibrosis in subjects that do not have artificially induced lung fibrosis (i.e. bleomycin-induced lung fibrosis in rats), human subjects, or subjects who are not vitamin A deficient.

There is an urgent need, therefore, to provide effective therapies for pulmonary fibrosis.

The applicant has recognised that vitamin A may be used to inhibit scar tissue formation in the lungs, thereby reducing damage caused to the lungs as a result of fibrosis and potentially allowing enhanced replacement of injured cells by cells of the same type in the regenerative phase. Vitamin A may thus be used in the effective prevention, treatment, or amelioration of pulmonary fibrosis.

According to the invention there is provided vitamin A for use as a pharmaceutically active agent in the prevention, treatment, or amelioration of pulmonary fibrosis in a subject, wherein the subject is not vitamin A deficient.

There is also provided according to the disclosure use of vitamin A in the manufacture of a medicament for the prevention, treatment, or amelioration of pulmonary fibrosis.

There is further provided according to the disclosure a method of preventing, treating, or ameliorating pulmonary fibrosis in a subject in need thereof, which comprises administering to the subject an effective amount of vitamin A.

In particular, vitamin A is able to prevent, treat, or ameliorate pulmonary fibrosis by inhibiting formation of pulmonary scar tissue.

It will be appreciated that use of a natural vitamin for the prevention, treatment, or amelioration of pulmonary fibrosis is particularly advantageous because of its known safety profile.

The pulmonary fibrosis may be a secondary effect of any of the following:
- an infection, including a viral infection, or a bacterial infection (such as tuberculosis)
- an autoimmune disease
- a connective tissue disease, such as rheumatoid arthritis, ankylosing spondylitis, systemic lupus erythematosus (SLE), or scleroderma
- a disease involving connective tissue, such as sarcoidosis and granulomatosis with polyangiitis
- administration of a medication, for example amiodarone, bleomycin (pingyangmycin), busulfan, methotrexate, apomorphine, and nitrofurantoin
- radiation therapy to the chest
- inhalation of an environmental or occupational pollutant, such as metals in asbestosis, silicosis or exposure to certain gases.
- hypersensitivity pneumonitis, most often resulting from inhaling dust contaminated with bacterial, fungal, or animal products

Optionally the pulmonary fibrosis is associated with a pulmonary infection. The pulmonary infection may be a viral, bacterial, or fungal pulmonary infection. Optionally the pulmonary infection is a chronic pulmonary infection.

The most common symptom of a chronic pulmonary infection is a persistent, severe cough (for example, lasting for more than three weeks). The sufferer will often bring up phlegm or mucus when coughing, and in the most severe cases, blood. Some patients with chronic pulmonary infections also experience some or all of the following symptoms (vary in severity from person to person): fever and sometimes sweats; a tight feeling across the chest, or sometimes sharp stabbing pain (pleurisy); shortness of breath which may involve wheezing; fatigue.

Examples of chronic pulmonary infections include: pneumonia, chronic bronchitis, influenza, the common cold, chronic sinusitis, rhinitis, Streptococcal pharyngitis, bronchiolitis, and bronchiectasis.

Causative agents of pulmonary infections that may be associated with pulmonary fibrosis include: *Bordetella pertussis* (whooping cough), Influenza A virus (for example, swine flu (H1N1), bird flu (H5N1)), influenza B virus, enterovirus, SARS coronavirus (SARS-CoV) (Severe acute respiratory syndrome, SARS), SARS coronavirus 2 (SARS-CoV-2) (Coronavirus Disease 2019, COVID-19), MERS coronavirus (MERS-CoV) (Middle East respiratory syndrome, MERS), *Histoplasma capsulatum* (Histoplasmosis), *Mycobacterium tuberculosis* (tuberculosis), *Blastomyces dermatitidis* (pulmonary blastomycosis).

Bacterial pneumonia is caused by *Streptococcus pneumoniae*, *Haemophilus influenza*, *Chlamydophila pneumoniae*, *Mycoplasma pneumoniae*; *Staphylococcus aureus; Moraxella catarrhalis*; and *Legionella pneumophila.* Viral pneumonia is caused by respiratory syncytial virus, parainfluenza, adenovirus, rhinoviruses, coronaviruses, influenza virus, respiratory syncytial virus (RSV), adenovirus, and parainfluenza. Fungal pneumonia is caused by *Histoplasma capsulatum, Blastomyces, Cryptococcus neoformans*, *Pneumocystis jiroveci* (pneumocystis pneumonia, or PCP), and *Coccidioides immitis.*

Viruses cause most cases of bronchitis and bronchiolitis. In community-acquired pneumonias, the most common bacterial agent is *Streptococcus pneumoniae.* Atypical pneumonias are cause by such agents as *Mycoplasma pneumoniae*, *Chlamydia spp*, Legionella, Coxiella burnetti and viruses. Nosocomial pneumonias and pneumonias in immunosuppressed patients have protean etiology with gram-negative organisms and staphylococci as predominant organisms.

Bronchiectasis may be associated with a range of bacterial, mycobacterial, and viral lung infections. Bacterial infections commonly associated with bronchiectasis include *P*. *aeruginosa*, *H. influenzae*, and *S. pneumoniae.* Nontuberculous mycobacteria infections such as *Mycobacterium avium* complex, and *Nocardia* infections have also been implicated.

Optionally the pulmonary infection is a viral pulmonary infection. Optionally the viral pulmonary infection is an Epstein-Barr virus (EBV), cytomegalovirus (CMV), human herpesvirus 7 (HHV-7), or human herpesvirus 8 (HHV-8) infection. Optionally the viral pulmonary infection is a coronavirus pulmonary infection, such as a severe acute respiratory syndrome coronavirus 2 (SARS-CoV-2) pulmonary infection.

Optionally the pulmonary fibrosis is idiopathic pulmonary fibrosis (IPF).

The term "pulmonary fibrosis" is used herein to include pulmonary fibrosis occurring in interstitial lung disease. Interstitial lung disease (ILD), or diffuse parenchymal lung disease (DPLD), is a group of lung diseases affecting the interstitium (the tissue and space around the alveoli (air sacs of the lungs). It concerns alveolar epithelium, pulmonary capillary endothelium, basement membrane, and perivascular and perilymphatic tissues. It may occur when an injury to the lungs triggers an abnormal healing response. Ordinarily, the body generates just the right amount of tissue to repair damage, but in interstitial lung disease, the repair process goes awry and the tissue around the air sacs (alveoli) becomes scarred and thickened. This makes it more difficult for oxygen to pass into the bloodstream. The average rate of survival for someone with this disease is currently between 3 and 5 years. Prolonged ILD may result in pulmonary fibrosis.

Idiopathic interstitial pneumonia is the term given to ILDs with an unknown cause. They represent the majority of cases of interstitial lung diseases (up to two-thirds of cases). They were subclassified by the American Thoracic Society in 2002 into 7 subgroups: Idiopathic pulmonary fibrosis (IPF): the most common subgroup; Desquamative interstitial pneumonia (DIP); Acute interstitial pneumonia (AIP): also known as Hamman-Rich syndrome; Nonspecific interstitial pneumonia (NSIP); Respiratory bronchiolitis-associated interstitial lung disease (RB-ILD); Cryptogenic organizing pneumonia (COP): also known as Bronchiolitis Obliterans Organizing Pneumonia (BOOP); Lymphoid interstitial pneumonia (LIP).

Secondary ILDs are those diseases with a known etiology, including:
Connective tissue and Autoimmune diseases:
   - Sarcoidosis;
   - Rheumatoid arthritis;
   - Systemic lupus erythematosus;
   - Systemic sclerosis;
   - Polymyositis;
   - Dermatomyositis;
   - Antisynthetase syndrome
Inhaled substances:
   Inorganic
      - Silicosis
      - Asbestosis
      - Berylliosis
      - Industrial printing chemicals (e.g. carbon black, ink mist)
   Organic
      - Hypersensitivity pneumonitis (Extrinisic allergic alveolitis)
   Drug-induced:
      - Antibiotics
      - Chemotherapeutic drugs
      - Antiarrhythmic agents
   Infection:
      - Coronavirus disease 2019 (COVID-19)
      - Atypical pneumonia
      - Pneumocystis pneumonia (PCP)
      - Tuberculosis
      - Chlamydia trachomatis
      - Respiratory Syncytial Virus
   Malignancy:
      - Lymphangitic carcinomatosis
   Predominately in children:
      - Diffuse developmental disorders
      - Growth abnormalities deficient alveolarisation
      - Infant conditions of undefined cause
      - ILD related to alveolar surfactant region

Vitamin A is the name of a group of fat-soluble retinoids, including retinol, retinal, and retinyl esters. There are two different categories of vitamin A. The first category, preformed vitamin A, comprises retinol and its esterified form, retinyl ester. The second category, provitamin A, comprises provitamin A carotenoids such as alpha-carotene, beta-carotene and beta-cryptoxanthin. Both retinyl esters and provitamin A carotenoids are converted to retinol, which is oxidized to retinal and then to retinoic acid. Both provitamin A and preformed vitamin A are known be metabolized intracellularly to retinal and retinoic acid, the bioactive forms of vitamin A.

Vitamin A for use according to the invention may be an isolated form of vitamin A. An isolated form of vitamin A is any form of vitamin A found in the diet or a metabolized form thereof. For example, vitamin A may be isolated from fish liver oil. Vitamin A may comprise a preformed vitamin A such as retinol or a retinyl ester. Retinyl esters include retinyl acetate and retinyl palmitate. Vitamin A may comprise a provitamin A, such as a provitamin A carotenoid including alpha-carotene, beta-carotene or beta-cryptoxanthin. Vitamin A may comprise a bioactive form of vitamin A such as retinal or retinoic acid.

Vitamin A is available for human consumption in multivitamins and as a stand-alone supplement, often in the form of retinyl acetate or retinyl palmitate. A portion of the vitamin A in some supplements is in the form of beta-carotene and the remainder is preformed vitamin A; others contain only preformed vitamin A or only beta-carotene. Supplement labels usually indicate the percentage of each form of the vitamin. The amounts of vitamin A in stand-alone supplements range widely. Multivitamin supplements typically contain 2,500 to 10,000 international units (IU) vitamin A, often in the form of both retinol and beta-carotene.

Vitamin A is listed on food and supplement labels in international units (IUs). However, Recommended Dietary Allowance (RDA) (average daily level of intake sufficient to meet the nutrient requirements of nearly all (97%-98%) healthy individuals) for vitamin A is given as micrograms (µg; mcg) of retinol activity equivalents (RAE) to account for the different bioactivities of retinol and provitamin A carotenoids (see Table 1). Because the body converts all dietary sources of vitamin A into retinol, 1 mcg of physiologically available retinol is equivalent to the following amounts from dietary sources: 1 mcg of retinol, 12 mcg of beta-carotene, and 24 mcg of alpha-carotene or beta-cryptoxanthin. From dietary supplements, the body converts 2 mcg of beta-carotene to 1 mcg of retinol.

Conversion rates between mcg RAE and IU are as follows:
- 1 IU retinol = 0.3 mcg RAE;
- 1 IU beta-carotene from dietary supplements = 0.15 mcg RAE;
- 1 IU beta-carotene from food = 0.05 mcg RAE; and
- 1 IU alpha-carotene or beta-cryptoxanthin = 0.025 mcg RAE.

An RAE cannot be directly converted into an IU without knowing the source(s) of vitamin A. For example, the RDA of 900 mcg RAE for adolescent and adult men is equivalent to 3,000 IU if the food or supplement source is preformed vitamin A (retinol). However, this RDA is also equivalent to 6,000 IU of beta-carotene from supplements, 18,000 IU of beta-carotene from food, or 36,000 IU of alpha-carotene or beta-cryptoxanthin from food. So a mixed diet containing 900 mcg RAE provides between 3,000 and 36,000 IU of vitamin A, depending on the foods consumed.

| Table 1: Recommended Dietary Allowances (RDAs) for Vitamin A | | | | |
|---|---|---|---|---|
| **Age** | **Male** | **Female** | **Pregnancy** | **Lactation** |
| 0-6 months* | 400 mcg RAE | 400 mcg RAE | | |
| 7-12 months* | 500 mcg RAE | 500 mcg RAE | | |
| 1-3 years | 300 mcg RAE | 300 mcg RAE | | |
| 4-8 years | 400 mcg RAE | 400 mcg RAE | | |
| 9-13 years | 600 mcg RAE | 600 mcg RAE | | |
| 14-18 years | 900 mcg RAE | 700 mcg RAE | 750 mcg RAE | 1,200 mcg RAE |
| 19-50 years | 900 mcg RAE | 700 mcg RAE | 770 mcg RAE | 1,300 mcg RAE |
| 51+ years | 900 mcg RAE | 700 mcg RAE | | |

| | | | | |
|---|---|---|---|---|
| * Adequate Intake (AI), equivalent to the mean intake of vitamin A in healthy, breastfed infants. Source: National Institutes of Health, Vitamin A, Fact Sheet for Health Professionals, as updated 5 October 2018 | | | | |

The Food and Nutrition Board (FNB) at the Institute of Medicine of the National Academies (formerly National Academy of Sciences) has established tolerable Upper Intake Level (UL) (maximum daily intake unlikely to cause adverse health effects) for preformed vitamin A that apply to both food and supplement intakes. The FNB based these ULs on the amounts associated with an increased risk of liver abnormalities in men and women, teratogenic effects, and a range of toxic effects in infants and children. The FNB has not established ULs for beta-carotene and other provitamin A carotenoids.

| Table 2: Tolerable Upper Intake Levels (ULs) for Preformed Vitamin A* | | | | |
|---|---|---|---|---|
| **Age** | **Male** | **Female** | **Pregnancy** | **Lactation** |
| 0-12 months | 600 mcg RAE (2,000 IU) | 600 mcg RAE (2,000 IU) | | |
| 1-3 years | 600 mcg RAE (2,000 IU) | 600 mcg RAE (2,000 IU) | | |
| 4-8 years | 900 mcg RAE (3,000 IU) | 900 mcg RAE (3,000 IU) | | |
| 9-13 years | 1,700 mcg RAE (5,667 IU) | 1,700 mcg RAE (5,667 IU) | | |
| 14-18 years | 2,800 mcg RAE (9,333 IU) | 2,800 mcg RAE (9,333 IU) | 2,800 mcg RAE (9,333 IU) | 2,800 mcg RAE (9,333 IU) |
| 19+ years | 3,000 mcg RAE (10,000 IU) | 3,000 mcg RAE (10,000 IU) | 3,000 mcg RAE (10,000 IU) | 3,000 mcg RAE (10,000 IU) |

| | | | | |
|---|---|---|---|---|
| Source: National Institutes of Health, Vitamin A, Fact Sheet for Health Professionals, as updated 5 October 2018 * These ULs, expressed in mcg and in IUs (where 1 mcg = 3.33 IU), only apply to products from animal sources and supplements whose vitamin A comes entirely from retinol or ester forms, such as retinyl palmitate. However, many dietary supplements (such as multivitamins) do not provide all of their vitamin A as retinol or its ester forms. For example, the vitamin A in some supplements consists partly or entirely of beta-carotene or other provitamin A carotenoids. In such cases, the percentage of retinol or retinyl ester in the supplement should be used to determine whether an individual's vitamin A intake exceeds the UL. For example, a supplement labeled as containing 10,000 IU of vitamin A with 60% from beta-carotene (and therefore 40% from retinol or retinyl ester) provides 4,000 IU of preformed vitamin A. That amount is above the UL for children from birth to 13 years but below the UL for adolescents and adults. | | | | |

Optionally vitamin A for use in the prevention, treatment, or amelioration of pulmonary fibrosis in a subject according to the invention comprises a high dose of vitamin A.

A high dose of vitamin A is considered to be a dose that exceeds a UL for the subject. Examples of high doses of vitamin A include: >10,000 IU to 100,000 IU vitamin A per day; about 25,000 to 50,000 IU per day; about 25,000 to 75,000 IU vitamin A per day; about 25,000 to 100,000 IU vitamin A per day; about 50,000 to 100,000 IU vitamin A per day; or about 75,000 to 100,000 IU vitamin A per day, in particular of preformed vitamin A.

Vitamin A may be administered to the subject once per day, twice per day, three times per day, four times per day, or five times per day.

Vitamin A may be administered to the subject for at least 3 days for example for at least a week, for at least a month, or for at least 6 months from the day of first administration to the subject.

Prolonged exposure to high doses of vitamin A may lead to hypervitaminosis A. Thus, it may be preferred to limit administration of high doses of vitamin A to the subject for up to 6 years, or up to 6 months, from the day of first administration to the subject.

Optionally for an adult human subject (>18 years old), the subject may be administered up to 100,000 IU vitamin A (in particular of preformed vitamin A) per day for up to 6 months. For example, >10,000 IU to 100,000 IU vitamin A per day; about 25,000 to 50,000 IU per day; about 25,000 to 75,000 IU vitamin A per day; about 25,000 to 100,000 IU vitamin A per day; about 50,000 to 100,000 IU vitamin A per day; or about 75,000 to 100,000 IU vitamin A per day (in particular of preformed vitamin A) for up to 6 months.

Optionally for an adult human subject (>18 years old), the subject may be administered up to 25,000 IU vitamin A per day (in particular of preformed vitamin A) for up to 6 years. For example, >10,000 IU to 25,000 IU vitamin A per day (in particular of preformed vitamin A) for up to 6 years.

Optionally the subject is administered up to 50% (for example >10% to 50%, or 25% to 50%) of a maximum safe dose of vitamin A (in particular of preformed vitamin A) for the subject per day.

For example, a maximum safe dose of vitamin A (in particular of preformed vitamin A) per day for an adult human subject may be 100,000 IU vitamin A (in particular of preformed vitamin A).

Optionally the subject is a mammalian subject. Optionally the subject is not a rat. Optionally the subject is a human subject.

The subject is not vitamin A deficient.

Plasma retinol levels are typically measured to assess vitamin A status. However, plasma retinol levels are under tight hepatic homeostatic control and do not decline until vitamin A concentration in the liver is almost depleted (critical liver concentration ≤20µg g⁻¹ of liver). Liver vitamin A reserves can be measured indirectly through the relative dose-response test (McLaren, D.S.; Kraemer, K. Manual on Vitamin Deficiency Disorders (VADD), 3rd ed.; Sight and Life Press:Basel, Switzerland, 2012; ISBN 978-3-906412-58-0), which is considered the "gold standard" indicator of whole-body vitamin A status. However, for clinical purposes, plasma retinol levels alone are sufficient and commonly used for documenting significant deficiency of vitamin A. The physiological plasma concentration of vitamin A is 1-2 µmol/L and, according to the World Health Organization, values of serum retinol concentrations below a cut-off of 0.70 µmol/L (or 20 µg/dL) represent biochemical vitamin A deficiency (VAD), and values lower than 0.35 µmol/L are indicative of severe deficiency and associated with numerous clinical manifestations.

Optionally the subject has a serum retinol concentration of at least 0.7 µmol/L.

Optionally the subject has a plasma concentration of vitamin A of 1-2 µmol/L.

Optionally the vitamin A is to be administered to the subject at a dose that results in a plasma concentration of vitamin A in excess of 2 µmol/L.

Optionally the subject has not been administered bleomycin.

Optionally the subject does not have bleomycin-induced lung fibrosis.

It will be appreciated that vitamin A should preferably be administered to the subject as soon as possible after the subject has been diagnosed as:
- being at risk of developing pulmonary fibrosis;
- having pulmonary fibrosis; or
- having a disease or condition (for example, an ILD) in which pulmonary fibrosis is a secondary effect.

Optionally vitamin A is administered within a month, within a week, or within a day of the diagnosis. Optionally vitamin A is administered with a week of the diagnosis.

Beneficial effects of treatment with vitamin A may also be observed when treatment is initiated many months or even years after diagnosis. Thus, optionally vitamin A may be administered months, years or even decades after diagnosis, for example within six months, a year, or a decade, or within twenty, thirty, forty, fifty, or sixty years of the diagnosis.

Pulmonary fibrosis may be diagnosed using any suitable techniques known to the skilled person. Examples (which may be used alone or in combination) include: X-rays; high-resolution computed tomography (HRCT) scans; semi-quantitative computed tomography (the most pertinent computed tomography patterns of fibrotic ILD are reticulation, traction, bronchiectasis, honeycombing and ground-glass opacification); computed tomography-derived quantitative lung fibrosis measures (quantitative CT); clinical markers (for example, dyspnoea and cough); physiological markers (for example, forced vital capacity (FVC) and, to a lesser extent, diffusing capacity of the lung for carbon monoxide (DLCO)); lung biopsy (usually by a small incision through the ribs with a thoracoscope); serum biomarkers; a pulmonary function test (using a device to measure breathing capacity), an oxygen desaturation study (the patient walks for almost 6 minutes while their oxygen level is measured through a probe attached to the finger or the forehead), other laboratory tests (for example, to rule out other diseases), including autoantibody tests, full blood count, electrolytes, creatinine levels, liver function tests, arterial blood gas.

Diagnosis of IPF is discussed in: Christe et al. ("Computer-Aided Diagnosis of Pulmonary Fibrosis Using Deep Learning and CT Images", Invest Radiol. 2019 Oct; 54(10): 627-632); Robbie et al., ("Evaluating disease severity in idiopathic pulmonary fibrosis", Eur Respir Rev 2017; 26: 170051); Martinez et al. ("The diagnosis of idiopathic pulmonary fibrosis: current and future approaches", Lancet Respir Med. 2017 Jan; 5(1): 61-71); Raghu et al. ("Diagnosis of idiopathic pulmonary fibrosis. An official ATS/ERS/JRS/ALAT clinical practice guideline", Am J Respir Crit Care Med. 2018 Sep 1;198(5):e44-e68); Nakamura and Suda ("Idiopathic Pulmonary Fibrosis: Diagnosis and Clinical Manifestations", Clin Med Insights Circ Respir Pulm Med. 2015; 9(Suppl 1): 163-171); Lynch et al. ("High-Resolution Computed Tomography in Idiopathic Pulmonary Fibrosis", Am J Respir Crit Care Med, Vol 172. pp 488-493, 2005).

It may be determined whether administration of vitamin A according to the invention has prevented, treated, or ameliorated pulmonary fibrosis in a subject by any technique known to the skilled person. Examples of suitable techniques (which may be used alone or in combination) include those referred to above, in particular X-rays, HRCT scans, semi-quantitative CT, quantitative CT, clinical markers, physiological markers, serum biomarkers, a lung biopsy, a pulmonary function test, or an oxygen desaturation study. Serum biomarkers, semi-quantitative CT, and/or quantitative CT may be particularly useful measures (see Robbie *et al.*, 2017)*.*

Effective treatment or amelioration of pulmonary fibrosis may include any slowing of the rate of progression of pulmonary fibrosis observed in the subject prior to administration of vitamin A (including, for example, any slowing of the rate of formation of pulmonary scar tissue), or any slowing of the rate of deterioration of lung function observed in the subject prior to administration of vitamin A.

The vitamin A may be administered to a subject by any suitable route. Examples include systemic administration, for example orally or intravenously. Optionally the vitamin A is administered to a subject by inhalation.

There is also provided according to the invention a pharmaceutical composition for oral administration, for use in the prevention, treatment, or amelioration of pulmonary fibrosis in a subject, which comprises Vitamin A as a pharmaceutically active agent, and a pharmaceutically acceptable plant oil, optionally wherein the plant oil comprises a coconut oil and/or wherein the pharmaceutical composition comprises a unit dose of vitamin A, wherein the unit dose comprises >10,000 IU to 100,000 IU vitamin A.

Optionally a pharmaceutical composition of the disclosure comprises a unit dose of vitamin A, wherein the unit dose comprises up to 100,000 IU vitamin A, for example >10,000 IU to 100,000 IU vitamin A; about 25,000 to 50,000 IU per day; about 25,000 to 75,000 IU vitamin A per day; 25,000 to 100,000 IU vitamin A; 50,000 to 100,000 IU vitamin A; or 75,000 to 100,000 IU vitamin A (in particular of preformed vitamin A).

There is also provided according to the disclosure a sterile sachet comprising a pharmaceutical composition of the invention.

There is further provided according to the disclosure a package comprising a plurality of separate unit doses of vitamin A, wherein each unit dose comprises a pharmaceutical composition of the invention.

A package of the disclosure may comprise at least 7 unit doses, at least 30 unit doses, or at least 100 unit doses of vitamin A.

Vitamin A of a pharmaceutical composition of the invention may comprise any combination of vitamin A described previously.

Use of vitamin A in accordance with the invention may be particularly effective for the treatment of older subjects. For example, a human subject may be at least 18 years old, at least 25 years old, at least 30 years old, at least 40 years old, or at least 50 years old.

The vitamin A can be incorporated into a variety of formulations for therapeutic administration, more particularly by combination with appropriate, pharmaceutically acceptable carriers, pharmaceutically acceptable diluents, or other pharmaceutically acceptable excipients, and can be formulated into preparations in solid, semi-solid, liquid or gaseous forms, such as tablets, capsules, powders, granules, ointments, solutions, suppositories, injections, inhalants and aerosols as appropriate.

Optionally the vitamin A is in solid form. Optionally the vitamin A is not in an organic solution. Optionally the vitamin A is not encapsulated by, or attached to a microparticle. Optionally the vitamin A is not encapsulated by, or attached to a nanoparticle.

Vitamin A can be administered in the form of a pharmaceutically acceptable salt. It can also be used alone or in appropriate association, as well as in combination, with other pharmaceutically active compounds. Optionally vitamin A is administered with an antibiotic agent, an anti-viral agent, or an anti-fungal agent. Optionally vitamin A is the only non-cellular, non-antibiotic, active agent administered.

The following methods and excipients are merely exemplary and are in no way limiting.

For oral preparations, vitamin A can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

Vitamin A can be formulated into preparations for injection by dissolving, suspending or emulsifying in an aqueous or nonaqueous solvent, such as vegetable or other similar oils, propylene glycol, synthetic aliphatic acid glycerides, injectable organic esters (e.g., ethyl oleate), esters of higher aliphatic acids or propylene glycol; and if desired, with conventional additives such as solubilizers, isotonic agents, suspending agents, emulsifying agents, stabilizers and preservatives. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Furthermore, a pharmaceutical composition of the present disclosure can comprise further agents such as dopamine or psychopharmacologic drugs, depending on the intended use of the pharmaceutical composition.

Pharmaceutical compositions are prepared by mixing Vitamin A having the desired degree of purity with optional pharmaceutically acceptable carriers, other excipients, stabilizers, surfactants, buffers and/or tonicity agents. Acceptable carriers, other excipients and/or stabilizers are nontoxic to recipients at the dosages and concentrations employed, and may include buffers such as phosphate, citrate, and other organic acids; antioxidants including ascorbic acid, glutathione, cysteine, methionine and citric acid; preservatives (such as ethanol, benzyl alcohol, phenol, m-cresol, p-chlor-m-cresol, methyl or propyl parabens, benzalkonium chloride, or combinations thereof); amino acids such as arginine, glycine, ornithine, lysine, histidine, glutamic acid, aspartic acid, isoleucine, leucine, alanine, phenylalanine, tyrosine, tryptophan, methionine, serine, proline and combinations thereof; monosaccharides, disaccharides and other carbohydrates; low molecular weight (less than about 10 residues) polypeptides; proteins, such as gelatin or serum albumin; chelating agents such as EDTA; sugars such as trehalose, sucrose, lactose, glucose, mannose, maltose, galactose, fructose, sorbose, raffinose, glucosamine, N-methylglucosamine, galactosamine, and neuraminic acid; and/or non-ionic surfactants such as Tween, Brij Pluronics, Triton-X, or polyethylene glycol (PEG).

The pharmaceutical composition can be in a liquid form, a lyophilized form or a liquid form reconstituted from a lyophilized form, wherein the lyophilized preparation is to be reconstituted with a sterile solution prior to administration. The standard procedure for reconstituting a lyophilized composition is to add back a volume of pure water (typically equivalent to the volume removed during lyophilization); however solutions comprising antibacterial agents can be used for the production of pharmaceutical compositions for parenteral administration; see also Chen (1992) Drug Dev Ind Pharm 18, 1311-54.

An aqueous formulation can be prepared in a pH-buffered solution, e.g., at pH ranging from about 4.0 to about 7.0, or from about 5.0 to about 6.0, or alternatively about 5.5. Examples of buffers that are suitable for a pH within this range include phosphate-, histidine-, citrate-, succinate-, acetate-buffers and other organic acid buffers. The buffer concentration can be from about 1 mM to about 100 mM, or from about 5 mM to about 50 mM, depending, e.g., on the buffer and the desired tonicity of the formulation.

A tonicity agent can be included in the formulation to modulate the tonicity of the formulation. Exemplary tonicity agents include sodium chloride, potassium chloride, glycerin and any component from the group of amino acids, sugars as well as combinations thereof. In some embodiments, the aqueous formulation is isotonic, although hypertonic or hypotonic solutions can be suitable. The term "isotonic" denotes a solution having the same tonicity as some other solution with which it is compared, such as a physiological salt solution or serum. Tonicity agents can be used in an amount of about 5 mM to about 350 mM, e.g., in an amount of 100 mM to 350 nM.

A surfactant can also be added to the formulation to reduce aggregation and/or minimize the formation of particulates in the formulation and/or reduce adsorption. Exemplary surfactants include polyoxyethylensorbitan fatty acid esters (Tween), polyoxyethylene alkyl ethers (Brij), alkylphenylpolyoxyethylene ethers (Triton-X), polyoxyethylene-polyoxypropylene copolymer (Poloxamer, Pluronic), and sodium dodecyl sulfate (SDS). Examples of suitable polyoxyethylenesorbitan-fatty acid esters are polysorbate 20, (sold under the trademark Tween 20^{™}) and polysorbate 80 (sold under the trademark Tween 80^{™}). Examples of suitable polyethylene-polypropylene copolymers are those sold under the names Pluronic^{®} F68 or Poloxamer 188^{™}. Examples of suitable Polyoxyethylene alkyl ethers are those sold under the trademark Brij^{™}. Exemplary concentrations of surfactant can range from about 0.001% to about 1% w/v.

A lyoprotectant can also be added in order to protect a labile active ingredient against destabilizing conditions during the lyophilization process. For example, known lyoprotectants include sugars (including glucose and sucrose); polyols (including mannitol, sorbitol and glycerol); and amino acids (including alanine, glycine and glutamic acid). Lyoprotectants can be included in an amount of about 10 mM to 500 nM.

In some embodiments, a subject formulation includes one or more of the above-identified agents (e.g., a surfactant, a buffer, a stabilizer, a tonicity agent) and is essentially free of one or more preservatives, such as ethanol, benzyl alcohol, phenol, m-cresol, p-chlor-m-cresol, methyl or propyl parabens, benzalkonium chloride, and combinations thereof. In other embodiments, a preservative is included in the formulation, e.g., at concentrations ranging from about 0.001 to about 2% (w/v).

Unit dosage forms for oral administration such as syrups, elixirs, and suspensions can be provided wherein each dosage unit, for example, teaspoonful, tablespoonful, or tablet contains a predetermined amount of the active agent (i.e. vitamin A). Similarly, unit dosage forms for injection or intravenous administration can comprise vitamin A in a composition as a solution in sterile water, normal saline or another pharmaceutically acceptable carrier.

The term "unit dosage form," as used herein, refers to physically discrete units suitable as unitary dosages for human or animal subjects, each unit containing a predetermined quantity of vitamin A, calculated in an amount sufficient to produce the desired effect in association with a pharmaceutically acceptable diluent, carrier or vehicle.

Vitamin A can be administered as an injectable formulation. Typically, injectable compositions are prepared as liquid solutions or suspensions; solid forms suitable for solution in, or suspension in, liquid vehicles prior to injection can also be prepared.

Suitable excipient vehicles are, for example, water, saline, dextrose, glycerol, ethanol, or the like, and combinations thereof. In addition, if desired, the vehicle can contain minor amounts of auxiliary substances such as wetting or emulsifying agents or pH buffering agents. Actual methods of preparing such dosage forms are known, or will be apparent, to those skilled in the art. See, e.g., Remington's Pharmaceutical Sciences, Mack Publishing Company, Easton, Pennsylvania, 17th edition, 1985. The composition or formulation to be administered will, in any event, contain a quantity of vitamin A adequate to achieve the desired state in the subject being treated.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

There is also provided according to the disclosure a pharmaceutical composition for inhalation, which comprises Vitamin A and a pharmaceutically acceptable carrier, excipient, or diluent.

Inhalable formulations are well known to the skilled person. Suitable examples are described in Hadiwinoto et al., "A review on recent technologies for the manufacture of pulmonary drugs", Therapeutic Delivery, Vol. 9, No. 1

The required aerosol size to deliver drugs to the whole lung involves an aerodynamic diameter < ~5µm. For delivery to the alveolar epithelium, particles with an even smaller size, for example, aerodynamic diameter < ~3µm, are required (Newman, "Drug delivery to the lungs: challenges and opportunities", Ther. Deliv. (2017) 8(8), 647-661).

Nanoparticles, microparticles, liposomes, powder, and microemulsions are commonly employed drug delivery carriers for pulmonary delivery (see Thakur et al., (2020) Patented therapeutic drug delivery strategies for targeting pulmonary diseases, Expert Opinion on Therapeutic Patents, 30:5, 375-387).

Optionally an excipient for a pharmaceutical composition of the disclosure for inhalation is selected from the group consisting of sugars and saccharides, preferably inhalation grade
lactose, preferably alpha monohydrate lactose in the form of crystalline lactose, milled lactose or micronized lactose.

Optionally a pharmaceutical composition of the disclosure for inhalation comprises particles having an aerodynamic diameter of 0.5 to 10µm.

Inhaled drug delivery is achieved using four principal technologies: dry powder inhalers, metered-dose inhalers, nebulisers and liquid inhalers. Suitable devices for administration of a pharmaceutical composition of the disclosure for inhalation are well-known to the skilled person. Examples are described in the following publications:
- Brunaugh A.D., Smyth H.D.C., Williams III R.O. (2019) Pulmonary Drug Delivery. In: Essential Pharmaceutics. AAPS Introductions in the Pharmaceutical Sciences. Springer, Cham, pp 163-181;
- Lalan M., Tandel H., Lalani R., Patel V., Misra A. (2019) Inhalation Drug Therapy: Emerging Trends in Nasal and Pulmonary Drug Delivery. In: Misra A., Shahiwala A. (eds) Novel Drug Delivery Technologies. Springer, Singapore
- Lexmond A., Forbes B. (2016) Drug Delivery Devices for Inhaled Medicines. In: Page C., Barnes P. (eds) Pharmacology and Therapeutics of Asthma and COPD. Handbook of Experimental Pharmacology, vol 237. Springer, Cham (pp 265-280);
- Ibrahim et al., "Inhalation drug delivery devices: technology update", Med Devices (Auckl). 2015; 8: 131-139.

Administration by inhalation may have advantages over systemic administration, including a more rapid onset of action, an increased therapeutic effect, and, depending on the agent inhaled, reduced systemic side effects since the required local concentration in the lungs can be obtained with a lower dose.

There is also provided according to the disclosure a dry powder inhaler, a metered-dose inhaler, a nebuliser, or a liquid inhaler, comprising a pharmaceutical composition of the disclosure for inhalation.

Ranges may be expressed herein as from "about" one particular value, and/or to another particular value. When such a range is expressed, another embodiment includes from the one particular value and/or to the other particular value. Similarly, when values are expressed as approximations, by the use of the antecedent "about", it will be understood that the particular value forms another embodiment.

Wherever the term "vitamin A" is used herein this includes reference to "vitamin A, or a pharmaceutically acceptable salt thereof".

Embodiments of the invention are now described, by way of example only, with reference to the accompanying drawings in which:
Figure 1 shows a schematic view of IPF pathogenesis:
   Repeated injuries over time lead to maladaptive repair process, characterized by AEC2s apoptosis, proliferation and epithelium-mesenchymal cross-talk (a) and following fibroblasts, myofibroblasts proliferation and accumulation of extracellular matrix (b). (abbreviations: CCL2: chemokine C-C motif ligand 2; CXCL12: C-X-C motif chemokine 12; FGF: fibroblast growth factor; PAI-1: plasminogen activator inhibitor 1; PAI-2: plasminogen activator inhibitor 2; PDGF: platelet-derived growth factor; TGF-β1: Transforming Growth Factor-Beta 1; TNF-α: tumor necrosis factor-alpha; VEGF: vascular endothelial growth factor). From Sgalla, et al. Idiopathic pulmonary fibrosis: pathogenesis and management. Respir Res 19, 32 (2018), distributed under the terms of the Creative Commons Attribution 4.0 International License (http://creativecommons.org/licenses/by/4.0/) (original figure in colour).

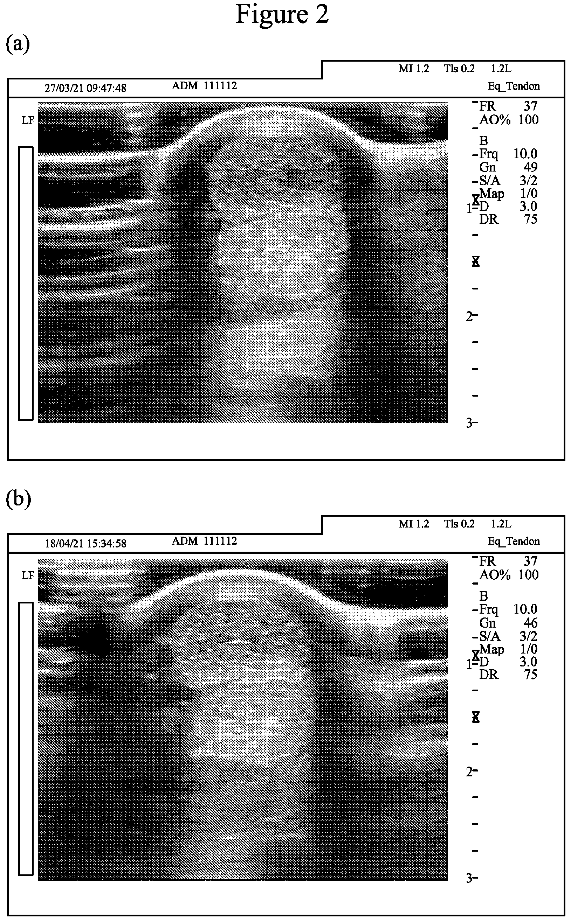

Figure 2 shows an ultrasonograph of a subtle core lesion in the lateral aspect of superficial digital flexor tendon (SDFT) with generalised surrounding tendonitis of a horse (ID 111112): (a) before administration of any pharmaceutical composition comprising vitamin A; and (b) after daily administration of a pharmaceutical composition comprising vitamin A for 14 days; and

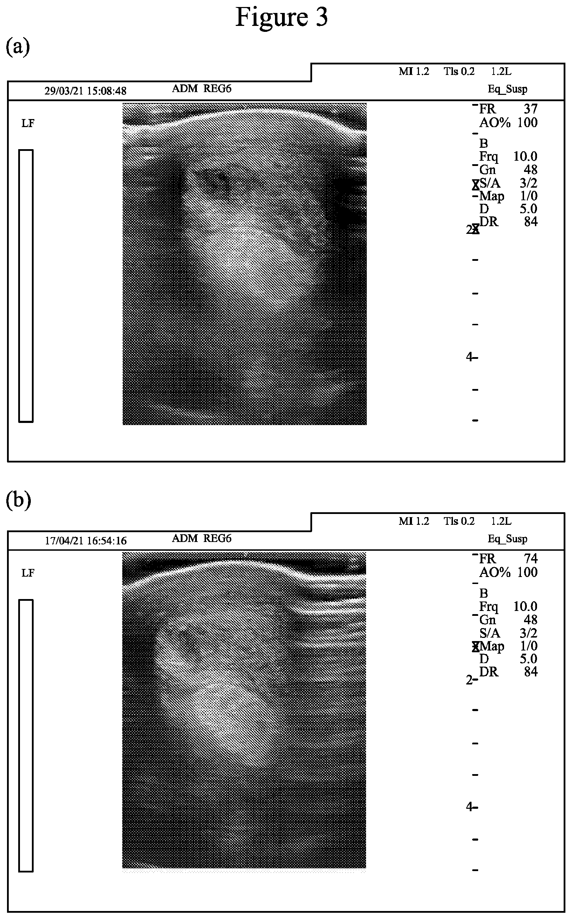

Figure 3 shows an ultrasonograph of a nasty SDFT core lesion in the medial aspect not quite involving paratenon for a horse (ID REG6): (a) before administration of any pharmaceutical composition comprising vitamin A; and (b) after daily administration of a pharmaceutical composition comprising vitamin A for 14 days.

### Example 1 - Pharmaceutical composition for oral administration

A pharmaceutical composition for oral administration to an adult human comprises a single unit dose of 50,000 IU Vitamin A in coconut oil. The pharmaceutical composition is provided in a sterile sachet.

### Example 2 - Pharmaceutical composition for oral administration

A pharmaceutical composition for oral administration to an adult human comprises a single unit dose of 25,000 IU/mL Vitamin A in coconut oil. The pharmaceutical composition is provided in a sterile sachet.

### Example 3

### Tendon Injury - recovery following treatment with Vitamin A

| | |
|---|---|
| **Patient** | Horse subject 1 (polo pony) |
| **Age** | 14 years |
| **Sex** | Mare |
| **Health status** | Good |

### Injury

| | |
|---|---|
| **Type of injury** | Tendon injury |
| **When the injury occurred** | 14 February 2019 |
| **Cause of the injury** | Hyperextension and/or blunt trauma |
| **Tissue(s) affected** | Tendon |
| **Impairments resulting from the injury** | Lameness and loss of athletic function |

### Treatment with conventional methods

Single dose 30mg dexamethasone IV to treat the initial acute inflammation followed by 1g phenylbutazone PO BID for 5 days. Ice applied to affected area for 15 minutes twice a day for 5 days.

### Extent of recovery following treatment with conventional methods

Recovery has progressed as expected with these types of lesions in horses. Moderate lameness, heat and pain response to firm digital palpation improved rapidly over the first 7 to 10 days. The horse then started a programme of incremental walking exercise.

### Treatment with Vitamin A

**How soon after the injury was treatment was treatment with Vitamin A initiated?**

Treatment with Vitamin A was initiated approximately 20 days after the initial injury.
**Type of Vitamin A administered**
   Retinyl palmitate (93.8% Retinol Equivalent)
**Route of administration**
   Oral
**Dose of Vitamin A per administration**
   750,000 IU
**Frequency of administration**
   Twice daily (1,500,000 IU per day)
**Period of administration**
   Continuing (at least 4 weeks)

### Recovery following treatment with Vitamin A

Functional recovery has been good considering the severity of the lesion. Ultrasonography has confirmed good reduction in the size of the lesion (notable reduction in size of anechoic pockets of interstitial haemorrhage). Horses are prey species and often do not show signs of overt pain or lameness even with severe lesions. It is not uncommon for such lesions to take between 12 to 18 months to heal.

### Example 4 - Treatment of equine tendon injury

This example describes the effect of a pharmaceutical composition comprising vitamin A in treating tendon injury in horses.

Tendon injuries result in the formation of a fibrovascular scar that never attains the characteristics of normal tendon. Tendon healing is characterised by the formation of fibrovascular scar tissue, as tendon has very little intrinsic regenerative capacity. The molecular mechanisms resulting in scar tissue formation after tendon injuries are not well understood (as reviewed in Schneider et al. Rescue plan for Achilles: Therapeutics steering the fate and functions of stem cells in tendon wound healing; Advanced Drug Delivery Reviews 129 2018 352-375). Briefly, in the first few days after injury a blood clot forms that serves as a preliminary scaffold for invading cells followed by a more robust vascular network which is essential for the survival of tenocytes engaged in the synthesis of new fibrous tissue. Thereafter, fibroblasts are recruited to the injured site and produce initially disorganised extracellular matrix components. Following this, a remodelling stage commences characterised by tissue changes resulting in a more fibrous appearance and eventually a scar-like tendon tissue can be observed.

Current biologic treatment strategies have not achieved tendon regeneration but include the use of extracellular matrix patches to provide a scaffold for new cell growth and differentiation (as reviewed in Galatz et al. Tendon Regeneration and Scar Formation: The Concept of Scarless Healing, J. Orthop. Res. 2015, 33(6) 823-831). Platelet rich plasma which comprises a multitude of growth factors normally involved in repair processes has also been investigated for use in tendon repair. However, there is no evidence that either strategy induces tendon regeneration. Tendon injuries are also a particular problem in horses.

Tendon injury has a similar pathophysiology to pulmonary fibrosis in that both diseases are characterised by excessive deposition of scar tissue. Evidence for an effective treatment of tendon injury (including evidence for inhibition of scar tissue formation following tendon injury) is considered to provide evidence for an effective treatment of pulmonary fibrosis (for example, through inhibition of scar tissue formation in pulmonary fibrosis).

### Pharmaceutical composition used:

Vitamin A palmitate (also known as preformed vitamin A, or retinyl palmitate) mixed with coconut oil to provide a final vitamin A concentration of 10,000 IU/ml.

### Administration of pharmaceutical composition:

Horses with tendon injury were orally administered vitamin A palmitate mixed with coconut oil, at a dose of 160,000 IU once per day for 14 days.

### Results:

Ultrasonographs of the lesions before administration of any pharmaceutical composition comprising vitamin A, and after daily administration of the composition for 14 days are shown for two different horses in Figures 2 and 3 (Figure 2: horse ID 111112; Figure 3: horse ID REG6).

Figure 2(a) (before any administration of the composition) shows a subtle core lesion in the lateral aspect of the superficial digital flexor tendon (SDFT) with generalised surrounding tendonitis. Figure 2(b) (after daily administration of the composition for 14 days) shows that the core lesion has filled in somewhat and is less hypoechoic, suggesting that something has "plugged" the hole. Whilst the nature and quality of the tissue in the lesion is hard to assess with ultrasonography, it certainly appears to be making positive progress after only two weeks.

Figure 3(a) (before any administration of the composition) shows a nasty SDFT core lesion in the medial aspect not quite involving paratenon. Again the lesion appears to be less hypoechoic on second scan (Figure 3(b) - after daily administration of the composition for 14 days) suggesting the lesion is filling in with tissue of some sort. Again, the nature and quality of the tissue filling this lesion is hard to assess with ultrasound, but the lesion appears to be making positive progress after only two weeks.

### Conclusions:

2 weeks in the field of equine chronic tendon/ligament injuries is a very short timescale and it is rare to see any significant change in these slow healing structures over such a short time period. In more acute injuries, there is a lot more early activity and ultrasonographic evidence of healing as the tendon responds to injury and the inflammatory cascade process commences.

The results presented here appear to show that vitamin A supplementation (by daily administration of the pharmaceutical composition) has had a positive effect on healing of the lesions after only 2 weeks.

## Claims

1. Vitamin A for use as a pharmaceutically active agent in the prevention, treatment, or amelioration of pulmonary fibrosis in a subject, wherein the subject is not vitamin A deficient.

2. Vitamin A for use according to claim 1, wherein the vitamin A inhibits formation of pulmonary scar tissue.

3. Vitamin A for use according to claim 1 or 2, wherein the pulmonary fibrosis is associated with a pulmonary infection, optionally wherein the pulmonary infection is a viral pulmonary infection, further optionally wherein the viral pulmonary infection is a severe acute respiratory syndrome coronavirus 2 (SARS-CoV-2) pulmonary infection.

4. Vitamin A for use according to any preceding claim, wherein the vitamin A comprises isolated vitamin A, a preformed vitamin A, such as a retinyl ester or retinol, a provitamin A carotenoid, or a bioactive form of vitamin A, such as retinal or retinoic acid.

5. Vitamin A for use according to any preceding claim for administration at a dose in excess of a Tolerable Upper Limit Intake Level (UL) for the subject.

6. Vitamin A for use according to any preceding claim wherein the subject is a human subject.

7. Vitamin A for use according to claim 6 for administration to the subject at a dose of >10,000 to 100,000 IU vitamin A per day,25,000-100,000, 50,000-100,000, or 75,000-100,000 IU vitamin A per day.

8. Vitamin A for use according to any preceding claim for administration to the subject once per day, twice per day, three times per day, four times per day, or five times per day.

9. Vitamin A for use according to any preceding claim for administration to the subject for at least 3 days, at least a week, at least a month, or at least 6 months from the day of first administration to the subject, optionally up to 6 years from the day of first administration to the subject.

10. Vitamin A for use according to any preceding claim for administration systemically, optionally orally or intravenously.

11. Vitamin A for use according to any preceding claim, for administration to the subject by inhalation.

12. Vitamin A for use according to any preceding claim, wherein the subject is not vitamin A deficient, optionally wherein the subject has a serum retinol concentration of at least 0.7 µmol/L, optionally wherein the subject has a plasma concentration of vitamin A of 1-2 µmol/L.

13. Vitamin A for use according to any preceding claim, for administration to the subject at a dose that results in a plasma concentration of vitamin A in excess of 2 µmol/L

14. Vitamin A for use according to any preceding claim, wherein the vitamin A is the only non-cellular, non-antibiotic, active agent to be administered to the subject.

15. A pharmaceutical composition for oral administration, for use in the prevention, treatment, or amelioration of pulmonary fibrosis in a subject, which comprises Vitamin A as a pharmaceutically active agent, and a pharmaceutically acceptable plant oil, optionally wherein the plant oil comprises coconut oil and/or wherein the pharmaceutical composition comprises a unit dose of vitamin A, wherein the unit dose comprises >10,000 IU to 100,000 IU vitamin A.

## Patentansprüche

1. Vitamin A zur Verwendung als pharmazeutischer Wirkstoff bei der Prävention, Behandlung oder Verbesserung von Lungenfibrose bei einem Patienten, wobei der Patient keinen Vitamin-A-Mangel hat.

2. Vitamin A zur Verwendung nach Anspruch 1, wobei das Vitamin A die Bildung von Lungennarbengewebe hemmt.

3. Vitamin A zur Verwendung nach Anspruch 1 oder 2, wobei die Lungenfibrose mit einer Lungeninfektion assoziiert ist, wobei die Lungeninfektion optional eine virale Lungeninfektion ist, wobei die virale Lungeninfektion ferner optional eine Lungeninfektion mit dem Schweres-akutes-Atemwegssyndrom-Coronavirus 2 (SARS-CoV-2) ist.

4. Vitamin A zur Verwendung nach einem der vorstehenden Ansprüche, wobei das Vitamin A isoliertes Vitamin A, ein vorgebildetes Vitamin A, wie ein Retinylester oder Retinol, ein Provitamin-A-Carotinoid oder eine bioaktive Form von Vitamin A, wie Retinal oder Retinsäure, umfasst.

5. Vitamin A zur Verwendung nach einem der vorstehenden Ansprüche zur Verabreichung in einer Dosis, die eine tolerierbare Obergrenze der Aufnahme (UL) für den Patienten überschreitet.

6. Vitamin A zur Verwendung nach einem der vorstehenden Ansprüche, wobei der Patient ein Mensch ist.

7. Vitamin A zur Verwendung nach Anspruch 6 zur Verabreichung an den Patienten in einer Dosis von >10.000 bis 100.000 IE Vitamin A pro Tag, 25.000-100.000, 50.000-100.000 oder 75.000-100.000 IE Vitamin A pro Tag.

8. Vitamin A zur Verwendung nach einem der vorstehenden Ansprüche zur Verabreichung an den Patienten einmal täglich, zweimal täglich, dreimal täglich, viermal täglich oder fünfmal täglich.

9. Vitamin A zur Verwendung nach einem vorstehenden Anspruch zur Verabreichung an den Patienten für mindestens 3 Tage, mindestens eine Woche, mindestens einen Monat oder mindestens 6 Monate ab dem Tag der ersten Verabreichung an den Patienten, optional bis zu 6 Jahre ab dem Tag der ersten Verabreichung an den Patienten.

10. Vitamin A zur Verwendung nach einem der vorstehenden Ansprüche zur systemischen, optional oralen oder intravenösen Verabreichung.

11. Vitamin A zur Verwendung nach einem der vorstehenden Ansprüche, zur Verabreichung an den Patienten durch Inhalation.

12. Vitamin A zur Verwendung nach einem der vorstehenden Ansprüche, wobei der Patient keinen Vitamin-A-Mangel hat, wobei der Patient optional eine Serum-Retinol-Konzentration von mindestens 0,7 µmol/l aufweist, wobei der Patient optional eine Plasmakonzentration von Vitamin A von 1-2 µmol/l aufweist.

13. Vitamin A zur Verwendung nach einem der vorstehenden Ansprüche zur Verabreichung an den Patienten in einer Dosis, die zu einer Plasmakonzentration von Vitamin A von mehr als 2 µmol/l führt

14. Vitamin A zur Verwendung nach einem der vorstehenden Ansprüche, wobei das Vitamin A der einzige nichtzelluläre, nicht-antibiotische Wirkstoff ist, der dem Patienten verabreicht wird.

15. Pharmazeutische Zusammensetzung zur oralen Verabreichung zur Verwendung bei der Prävention, Behandlung oder Verbesserung von Lungenfibrose bei einem Patienten, die Vitamin A als pharmazeutischen Wirkstoff und ein pharmazeutisch akzeptables Pflanzenöl umfasst, wobei das Pflanzenöl optional Kokosöl umfasst und/oder wobei die pharmazeutische Zusammensetzung eine Einheitsdosis von Vitamin A umfasst, wobei die Einheitsdosis >10.000 IE bis 100.000 IE Vitamin A umfasst.

## Revendications

1. Vitamine A pour utilisation comme agent pharmaceutiquement actif dans la prévention, le traitement ou l'amélioration de la fibrose pulmonaire chez un sujet, dans laquelle le sujet n'est pas carencé en vitamine A.

2. Vitamine A pour utilisation selon la revendication 1, dans laquelle la vitamine A inhibe la formation de tissu cicatriciel pulmonaire.

3. Vitamine A pour utilisation selon la revendication 1 ou 2, dans laquelle la fibrose pulmonaire est associée à une infection pulmonaire, facultativement dans laquelle l'infection pulmonaire est une infection pulmonaire virale, encore facultativement dans laquelle l'infection pulmonaire virale est une infection pulmonaire par le coronavirus 2 du syndrome respiratoire aigu sévère (SRAS-CoV-2).

4. Vitamine A pour utilisation selon l'une quelconque des revendications précédentes, dans laquelle la vitamine A comprend de la vitamine A isolée, une vitamine A préformée, telle qu'un ester de rétinyle ou du rétinol, un caroténoïde de provitamine A, ou une forme bioactive de vitamine A, telle que le rétinal ou l'acide rétinoïque.

5. Vitamine A pour utilisation selon l'une quelconque des revendications précédentes, pour une administration à une dose excédant un niveau d'apport limite supérieur (LS) tolérable pour le sujet.

6. Vitamine A pour utilisation selon l'une quelconque des revendications précédentes, dans laquelle le sujet est un sujet humain.

7. Vitamine A pour utilisation selon la revendication 6 pour une administration au sujet à une dose de > 10 000 à 100 000 UI de vitamine A par jour, 25 000 à 100 000, 50 000 à 100 000 ou 75 000 à 100 000 UI de vitamine A par jour.

8. Vitamine A pour utilisation selon l'une quelconque des revendications précédentes pour une administration au sujet une fois par jour, deux fois par jour, trois fois par jour, quatre fois par jour ou cinq fois par jour.

9. Vitamine A pour utilisation selon l'une quelconque des revendications précédentes pour une administration au sujet pendant au moins 3 jours, au moins une semaine, au moins un mois, ou au moins 6 mois à compter du jour de la première administration au sujet, facultativement jusqu'à 6 ans à compter du jour de la première administration au sujet.

10. Vitamine A pour utilisation selon l'une quelconque des revendications précédentes pour une administration systémique, facultativement par voie orale ou intraveineuse.

11. Vitamine A pour utilisation selon l'une quelconque des revendications précédentes, pour une administration au sujet par inhalation.

12. Vitamine A pour utilisation selon l'une quelconque des revendications précédentes, dans laquelle le sujet n'est pas carencé en vitamine A, facultativement dans laquelle le sujet présente une concentration sérique en rétinol d'au moins 0,7 µmol/L, facultativement dans laquelle le sujet présente une concentration plasmatique en vitamine A de 1-2 µmol/L.

13. Vitamine A pour utilisation selon l'une quelconque des revendications précédentes, pour une administration au sujet à une dose qui conduit à une concentration plasmatique de vitamine A excédant 2 µmol/L.

14. Vitamine A pour utilisation selon l'une quelconque des revendications précédentes, dans laquelle la vitamine A est le seul agent actif non cellulaire, non antibiotique, à administrer au sujet.

15. Composition pharmaceutique pour administration orale, pour utilisation dans la prévention, le traitement ou l'amélioration de la fibrose pulmonaire chez un sujet, qui comprend de la vitamine A en tant qu'agent pharmaceutiquement actif, et une huile végétale pharmaceutiquement acceptable, dans laquelle facultativement l'huile végétale comprend de l'huile de coco et/ou dans laquelle la composition pharmaceutique comprend une dose unitaire de vitamine A, dans laquelle la dose unitaire comprend >10 000 UI à 100 000 UI de vitamine A.
